# EUROPEAN PATENT APPLICATION

(11) **EP 0 923 945 A2**
(43) Date of publication of application: **23.06.1999**
(21) Application number: 98123450.3
(22) Date of filing: 11.12.1998
(51) Int. Cl.: A61L 2/04, A61L 2/08, A61L 2/20

(54) **Cross-linking and sterilization treatment for manufacturing polyethylene items with high-level tribological characteristics, particularly for biomedical applications**

(30) Priority: 16.12.1997 IT BO970722
(71) Applicant: Società per Azioni SAMO, 40057 Cadriano di Granarolo Emilia (Bologna) (IT)
(72) Inventor: Alfonsi, Stefano, 40131 Bologna (IT); Cigada, Alberta, 20213 Milano (IT); Chiesa, Roberto, 21040 Vedano Olona (IT); Tanzi, Maria Cristina, 20213 Milano (IT)
(74) Representative: Modiano, Guido, Dr.-Ing.

(57) **Abstract**

A cross-linking and sterilization treatment for manufacturing items made of polyethylene with high-level tribological characteristics, particularly for biomedical applications, which consists of: a first step, during which an intermediate bar or plate made of a material of the type known by the acronym UHMWPE is irradiated with a dose of high-energy radiation between 100 kGy and 600 kGy; a heat treatment step at a temperature between 130°C and 170°C for a time between 48 and 72 hours; a step for the machining of the bar or plate to form the item; and a final sterilization step performed by means of known methods which use ethylene oxide.

## Description

The present invention relates to a cross-linking and sterilization treatment for manufacturing polyethylene items with ultrahigh molecular weight (hereinafter referenced to with the acronym UHMWPE, which stands for Ultra-High Molecular Weight Polyethylene) with high-level tribological characteristics, particularly for biomedical applications.

UHMWPE is a material which is widely used in the manufacture of orthopedic prostheses and particularly for acetabular cups of hip prostheses and for the production of components for knee prostheses: its mechanical and tribological characteristics and its excellent biocompatibility make it suitable for manufacturing articular surfaces of orthopedic endoprostheses, which require high wear resistance and a low friction coefficient.

The use of UHMWPE for the articular components of orthopedic prostheses, however, is not free from problems, which are mainly linked to the occurrence of wear, as shown by an extensive number of cases and by exhaustive documentation: said wear often becomes apparent, through use, as a production of micrometric polymer particles which can lead to mobilization, i.e., to the gradual separation of the stem of the prosthesis from the femur or of the acetabular cup from the pelvis.

It is currently generally acknowledged that mobilization is induced by the particles loosened by the wear of articulations.

The structure and accordingly the wear-resistance of UHMWPE used for orthopedic applications changes considerably during molding and sterilization, which prosthetic components must undergo before implantation in the patient. In particular, the tribological behavior of UHMWPE can be influenced by oxidation and/or cross-linking during the molding and setting of the resin and most of all during sterilization, but also during storage and during use of the prosthesis.

Almost all UHMWPE prosthetic components are sterilized with gamma rays at a dose of approximately 25 kGy. This radiation always causes changes in the characteristics of the material. Ionizing radiation breaks the C-C bonds of the macromolecular chains of polyethylene and can accordingly create highly reactive free radicals. The behavior of these free radicals produced by radiation depends mainly on whether chemical species capable of reacting with them are present. If oxygen is present, said radicals tend to bond with it, terminating the chain and accordingly reducing the molecular weight of the material.

In order to improve the wear behavior of UHMWPE, the level of cross-liking has already been increased for a long time by using high doses (200-1500 kGy) of gamma or beta rays, and subsequent heat treatments have been performed to facilitate cross-linking after irradiation: however, no solution has been found to the problem of what happens to said cross-linked structure during a subsequent sterilization treatment if said treatment, as indeed occurs, is required.

The aim of the present invention is to obviate the cited drawbacks of the prior art, i.e., to provide a cross-linking and sterilization treatment for manufacturing items made of polyethylene with high-level tribological characteristics, particularly for biomedical applications, which minimizes the unwanted formation of polymer particles after implantation.

Within the scope of this aim, an object of the present invention is to provide a treatment which is simple, relatively easy to perform in practice, safe in use, effective in operation, and has a relatively low execution cost.

This aim, this object and others which will become apparent hereinafter are achieved by the present cross-linking and sterilization treatment for manufacturing items made of polyethylene with high-level tribological characteristics, particularly for biomedical applications, comprising: a first step, during which an intermediate bar or plate made of a material of the type known by the acronym UHMWPE is irradiated with a dose of high-energy radiation between 100 kGy and 600 kGy; a heat treatment step at a temperature preferably between 130°C and 170°C for a time preferably between 48 and 72 hours; a step for the machining of the bar or plate to form the item; and a final sterilization step performed by means of methods which use ethylene oxide.

Further characteristics and advantages of the present invention will become apparent from the following detailed description of a preferred but not exclusive embodiment of a cross-linking and sterilization treatment for manufacturing items made of polyethylene with high-level tribological characteristics, particularly for biomedical applications, according to the invention, illustrated only by way of non-limitative example with particular reference to the following example.

### EXAMPLE

-- A first step during which an intermediate bar or plate made of a material known by the acronym UHMWPE is irradiated with a dose of high-energy gamma radiation at 200 kGy in an air atmosphere;
-- a step consisting in heat treatment at a temperature of 130°C (far from the softening temperature) in vacuum for at least 48 hours;
-- a step consisting in machining the bar or plate with known machine tools to produce the item;
-- a step consisting in sterilization by means of known methods and devices which use ethylene oxide.

The irradiation is capable of breaking the long linear chains and activates them, creating free radicals which can react with the oxygen and/or produce cross-linking of the chains and can therefore be deactivated.

The dosage of the radiation, which can be of various kinds, cannot be lower than 100 kGy (no appreciable cross-linking is apparent below this value) and should not exceed 600 kGy (since no further improvement of the material is achieved above this value).

Irradiation can advantageously be performed in a nitrogen or argon atmosphere as well.

The heat treatment can be performed during or after irradiation at temperatures between 130°C and 170°C for a time period advantageously between 60 and 360 minutes, brings the material close to the softening temperature and stabilizes the cross-linked structure of the polyethylene. The increase in the mobility of the active chains achieved by applying heat energy facilitates the reaction of the free radicals, forming a cross-linked structure (with an actual increase in the molecular weight of the polymer). The heat treatment is performed in distilled water deoxygenated by means of repeated flushing with nitrogen.

Water deoxygenation is achieved by introducing nitrogen at a pressure preferably of 5-10 bar and bubbling it through in an autoclave which contains the bar of UHMWPE immersed in distilled water. After injecting the nitrogen in the autoclave, a valve is opened and the internal pressure is advantageously returned to 1 atmospheric bar. This procedure is repeated preferably for at least five cycles so that the oxygen dissolved in water is extracted due to the presence of the nitrogen at a higher pressure.

Without this heat treatment, the active chains would terminate with oxygen or with any other molecules or atomic species (consequently reducing the molecular weight).

The final sterilization, performed with methods which use ethylene oxide, is essential for the satisfactory outcome of the treatment according to the invention.

If a conventional sterilization by irradiation with gamma or beta rays were performed, the problem of the activation of free radicals, solved earlier with the heat treatment, would reoccur, consequently reducing the molecular weight: sterilization by means of ethylene oxide instead has no effect from this point of view.

It has thus been observed that the invention achieves the intended aim and object.

The invention thus conceived is susceptible of numerous modifications and variations, all of which are within the scope of the inventive concept.

All the details may furthermore be replaced with other technically equivalent elements.

In practice, the indicated values and the intervention times may be any according to requirements without thereby abandoning the scope of the protection of the appended claims.

The disclosures in Italian Patent Application No. BO97A000722 from which this application claims priority are incorporated herein by reference.

## Claims

1. A cross-linking and sterilization treatment for manufacturing items made of polyethylene with high-level tribological characteristics, particularly for biomedical applications, characterized in that it comprises: a first step, during which an intermediate bar or plate made of a material of the type known by the acronym UHMWPE is irradiated with a dose of high-energy radiation between 100 kGy and 600 kGy; a heat treatment step; a step for the machining of the bar or plate to form the item; and a final sterilization step performed by means of ethylene oxide.

2. The treatment according to claim 1, characterized in that said irradiation is performed with gamma rays at 200 kGy.

3. The treatment according to claim 1 or 2, characterized in that said irradiation is performed in an air atmosphere.

4. The treatment according to claim 1, characterized in that said heat treatment step is performed at a temperature between 130°C and 170°C for a time between 48 and 72 hours.

5. The treatment according to claim 1, characterized in that said heat treatment is performed at 130°C.

6. The treatment according to claim 1, characterized in that said heat treatment is performed in distilled water which is deoxygenated by means of repeated cycles of low-pressure nitrogen bubbling.
